# EUROPEAN PATENT SPECIFICATION

(11) **EP 2 645 988 B1**
(45) Date of publication and mention of the grant of the patent: **11.01.2017**
(21) Application number: 11788473.4
(22) Date of filing: 29.11.2011
(51) Int. Cl.: A61K 8/81, A61K 8/84, A61Q 5/04

(54) **COMPOSITION FOR THE PERMANENT SHAPING OF HUMAN HAIR**
ZUSAMMENSETZUNG ZUR DAUERHAFTEN FORMUNG VON MENSCHLICHEM HAAR
COMPOSITION POUR LE FAÇONNAGE PERMANENT DE CHEVEUX HUMAINS

(30) Priority: 01.12.2010 EP 10015164
(43) Date of publication of application: 09.10.2013
(73) Proprietor: Kao Germany GmbH, 64297 Darmstadt (DE)
(72) Inventor: WOOD, Jonathan, 69469 Weinheim (DE); MEUSER, Alexandra, 63329 Egelsbach (DE); SCHNEIDER, Jörg, 64347 Griesheim (DE)
(74) Representative: Grit, Mustafa
(86) International application number: PCT/EP2011/071246
(87) International publication number: WO 2012/072616

(56) References cited:
- EP-A1- 2 177 245
- FR-A2- 2 465 478

## Description

The present invention concerns a composition for the permanent shaping of human hair used both for the permanent waving of human hair with an excellent waving effect as well as for the straightening of either naturally or chemically curled hair.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement, especially in permanent shaping of damaged to strongly damaged hair and in particular for permanent shaping hair streaks including parts with various damage level in length.

Use of polymers in permanent shaping compositions for hair has been known for many years. For examples in EP 797861 A1 permanent shaping compositions are disclosed comprising homopolymer of dimethyl diallyl ammonium chloride.

EP2177245
which has been published for our company, disclose permanent shaping compositions comprising copolymer of vinylpyrrolidone and quaternized vinylimidazole (Polyquaternium-87) and optionally additional cationic polymer including Polyquaternium-2. Further permanent hair shaping compositions comprising urea copolymers are known from FR2465478.

The present invention starts from the task of providing a composition for the permanent shaping of human hair with excellent waving and straightening performance. Hair waved or straightened with composition disclosed herein looks and feels natural upon touching by hand. For waved hair it is especially important that the hair has excellent elasticity and bounce.

Accordingly, the first object of the present invention is a composition for permanent shaping and/or straightening hair based on at least one reducing agent further comprising a first cationic polymer consisting of a copolymer of vinylpyrrolidone and vinylimidazole and homopolymer of dimethyl diallyl ammonium chloride and a second cationic polymer selected from urea copolymers. Vinylimidazole in the copolymer of first cationic polymer is not quaternised. Cationic charge density of the first cationic polymer is preferably between 3 and 4 mEq/g and particularly 3.7 mEq/g at pH 7.0.

Recently a cationic polymer has become commercially available under the trade name Luviquat Sensation from BASF with CTFA name Polyquaternium-87 which is the cationic polymer particularly suitable as the first cationic polymer for the compositions of the present invention.

Composition of the present invention comprises preferably a first cationic polymer at a concentration of 0.1 to 2.5%, preferably 0.2 to 2%, more preferably 0.25 to 1.5% and most preferably 0.25 to 1% by weight calculated to total composition.

Composition of the present invention comprises further a second cationic polymer selected from urea copolymers. Suitable and most preferred cationic polymer is Polyquaternium-2 commercially available under the trade name Mirapol A-15 from Rhodia.

Second cationic polymer is preferably comprised in the compositions of the present invention at a concentration of 0.1 to 2.5%, preferably 0.2 to 2%, more preferably 0.25 to 1.5% and most preferably 0.25 to 1% by weight calculated to total composition.

It has furthermore been find out that weight ratio of the first ad second cationic polymers play an important role in obtaining the best permanent shaping efficiency. In preferred embodiment of the present invention, first and second cationic polymers are comprise in the composition at a first to second cationic polymer ratio in the range of 5:1 to 1:5, preferably 3:1 to 1:3 and more preferably 2:1 to 1:2 and most preferably 1 :1.

It is furthermore preferred that total concentration of the first and the second cationic polymers in the compositions is in the range of 0.2 to 5%, preferably 0.2 to 2%, more preferably 0.25 to 1.5% and most preferably 0.25 to 1% by weight calculated to total composition.

The permanent shaping compositions according to the invention comprise at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 1.0 to 12.5%, more preferably 2.0 to 12.5% by weight, calculated to total composition as free thioglycolic acid as reference substance.

The permanent shaping compositions containing reducing agents can, if necessary, comprise alkalizing agents. Their quantity is dependent on the reducing agent and the desired pH-value of the composition. Reducing agent compositions preferably contain 0.1 % to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between about 6.5 and 10.5, preferably about 7 to 9.5.

Permanent shaping compositions according to the invention are suited for use both for the permanent waving, i.e. curling of human hair and for the straightening, i.e. smoothing thereof.

The viscosity best suited for the permanent shaping compositions according to the invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known **per se,** such as cellulose derivatives. Thickening may as well be realized by formulating a composition in form of an emulsion with the use of C₁₀-C₂₂-fatty alcohols, in mixture with long mono alkyl chain quaternary ammonium surfactants.

Permanent shaping compositions according to the present invention preferably comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 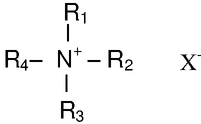 where R₁s a saturated or unsaturated, branched or non-branched alkyl chain with 8-24 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a saturated or unsaturated, branched or non-branched alkyl chain with 1-24 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are same or different lower alkyl chain with 1 to 4 carbon atoms which may be substituted with one or two hydroxyl group, and X is chloride, bromide or methosulfate.

Concentration of cationic surfactant is in the range from 0.05 % to 5 %, preferably 0.1 % to 2.5 % by weight, calculated to total composition.

Suitable long-chain quaternary ammonium compounds are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Further permanent shaping compositions of the present invention may comprise additional cationic polymer. Basically suitable are all cationic polymers listed under the generic name "Polyquaternium" in the CTFA International Cosmetic Ingredient Dictionary. Examples are Polyquaternium 4, Polyquaternium 5, Polyquaternium 6, Polyquaternium 7, Polyquaternium 10, Polyquaternium 11, Polyquaternium 16, Polyquaternium 22 and Polyquaternium 28, and Polyquaternium 39,
The cationic polymers also include the quaternized products of graft polymers from organopolysiloxanes and polyethyl oxazolines described in EP-A 524 612 and EP-A 640 643. Such polymer is known with its CTFA name Polysilicone-9.

Concentration of one or more additional cationic polymers is in the range from 0.05 % to 2.5 %, preferably 0.1 % to 1.5 % by weight, calculated to total composition. Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

Permanent shaping composition of the present invention can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 2.5% by weight calculated to total composition.

Another preferred compound in the permanent shaping composition of the present invention is silicone compounds and especially aminated silicones such as amodimethicone available from for example Dow Corning under the brand names Dow Corning 949 Emulsion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the permanent shaping compositions of the present invention is ubichinone of the formula 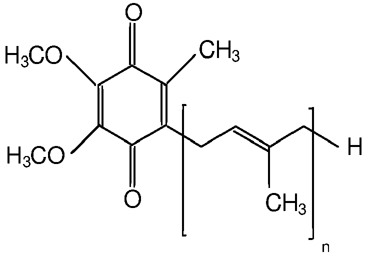 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

In another preferred embodiment of the present invention, composition comprises one or more fatty alcohol with a chain length of 12 to 24 C atoms. Concentration of one or more fatty alcohol is in the range of 1 to 15%, preferably 1 to 10%, more preferably 2 to 7.5 and most preferably 2 to 5% by weight calculated to total composition.

Suitable non limiting examples are lauryl alchohol, myristyl alcohol, cetyl alcohol, stearyl alcohol cetearyl alcohol, behenyl alcohol and their mixtures. Preferred are cetyl alcohol, stearyl alcohol cetearyl alcohol, behenyl alcohol and their mixtures. Particularly preferred are cetyl alcohol, stearyl alcohol and their 1 to 1, by weight, mixture cetearyl alcohol.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Ole-Fette-Wachse", 117 (1991), pages 81 to 87.

Composition of the present invention is used in a process for permanent waving wherein hair is washed or shampooed first and wound on the curlers, subsequently a reducing agent composition comprising at least one reducing agent, a first cationic polymer consisting of copolymer of vinylpyrrolidone and vinylimidazole and homopolymer of dimethyl diallyl ammonium chloride and a second cationic olymer selected fro urea copolymers is applied onto hair and after 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers removed from hair. In cases where additional conditioning composition is required this may as well be applied after finishing the process as described above.

In another process as described above the curlers are removed after rinsing off the reducing agent and before applying the oxidizing agent.

Further in another process, after rinsing off the reducing agent from hair, an intermediate treatment composition is applied onto hair and without rinsing off but after removing the excess amount of intermediate treatment with a towel, oxidizing composition is applied and at the end of the processing they are rinsed off from hair and curlers are removed from hair.

It has further been found out that the use of first cationic polymer consisting of a copolymer of vinylpyrrolidone and vinylimidazole and homopolymer of dimethyl diallyl ammonium chloride and a second cationic polymer selected from urea copolymers in the intermediate treatment composition improves the permanent shaping of hair as well in terms of curl appearance and natural look and feel of hair. Therefore, in another preferred form of the present invention, permanent shaping of hair is carried our with a process wherein hair is washed or shampooed first and subsequently a reducing agent comprising composition is applied onto hair and after 1 to 45 min, preferably 1 to 30 min of processing time, depending on the hair strength, rinsed off from hair with tap water and an intermediate treatment composition comprising a first cationic polymer consisting of a copolymer of vinylpyrrolidone and vinylimidazole and homopolymer of dimethyl diallyl ammonium chloride and a second cationic polymer selected from urea copolymers is applied and without rinsing off an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off. In cases where additional conditioning composition is required this may as well be applied after finishing the above process. In case of permanent waving hair in the above process curlers are put onto hair before application of reducing composition and taken off from hair before application of oxidizing composition or after application and processing of the oxidizing composition.

The intermediate treatment composition has a pH value between 2.5 to 6, preferably 3 to 5.5 and most preferably 3 to 5.

A straightening process may also be carried out in a different process wherein hair is washed and/or shampooed and dried and reducing composition comprising at least one reducing compound and a first cationic polymer consisting of a copolymer of vinylpyrrolidone and vinylimidazole and homopolymer of dimethyl diallyl ammonium chloride and a second cationic polymer selected from urea copolymers is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

The following examples are to illustrate the invention, but not to limit it.

### Example 1:

| | |
|---|---|
| Ammonium thioglycolate (60%) | 21.3 (% by wt |
| Ammonium hydrogen carbonate | 5.0 |
| 1,3- butylene gylcol | 3.0 |
| Polyquaternium-87* | 0.25 |
| Polyquaternium - 2 | 0.25 |
| PEG-40-Hydrogenated castor oil | 0.7 |
| Perfume | 0.4 |
| Ammonia, 25% | ad pH 8.3 |
| Water | ad 100.0 |

| | |
|---|---|
| * Cationic polymer used was Luviquat Sensation from BASF. | |

With this composition the hair was permanently waved for about 15 minutes, rinsed and neutralized for about 8 minutes with a customary 2.5% H₂0₂ composition. Homogeneous wave appearance was obtained. Exclusion of Polyquaternium 87 and Polyquaternium 2 resulted in less homogeneous perm appearance.

It has furthermore found out that the improved permanent waving efficiency with the two cationic polymers in combination was synergistic as improved curling efficiency observed when both copolymers comprised in the compositions was not simply sum of the effects observed with the compositions comprising one of the cationic polymers at the same total polymer concentration.

In all of the following examples, Luviquat Sensation from BASF was used as the first cationic polymer. And Mirapol A-15 is used as the second cationic polymer.

### Example 2:

| | |
|---|---|
| Ammonium thioglycolate (60%) | 20 (% by wt |
| Ammonium hydrogen carbonate | 4.0 |
| 1,3- butylene gylcol | 3.0 |
| Polyquaternium-87 | 0.25 |
| Polyquaternium - 2 | 0.25 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 15 minutes, rinsed and neutralized for about 8 minutes with a customary 2.5% H₂0₂ composition. Homogeneous wave appearance was obtained. Exclusion of Polyquaternium-87 and Polyquaternium-2 resulted in less homogeneous perm appearance.

### Example 3:

### Alkaline Permanent Wave for Damaged Hair

| | |
|---|---|
| Ammonium thioglycolate (60%) | 15.0 (% by wt.) |
| Ammonium hydrogen carbonate | 2.5 |
| Ceteth-20 | 0.7 |
| 1,3- butylene gylcol | 0.5 |
| Polyquaternium-87 | 0.5 |
| Polyquaternium-2 | 0.5 |
| Perfume | 0.4 |
| Ammonia, 25% | ad pH 8.0 |
| Water | ad 100.0 |

The permanent wave achieved with this composition was similar to the one obtained with the composition according to Example 1.

Exclusion of the Polyquaternium-87 and Polyquaternium-2 led to waves with substantially weaker contours.

### Example 4:

| | |
|---|---|
| Ammonium thioglycolate (60%) | 0.9 (% by wt.) |
| Cystein hydrochloride | 5.7 |
| Ammonium hydrogen carbonate | 1.5 |
| Acetylcystein | 0.7 |
| Cetrimonium chloride | 0.1 |
| 1,3- butylene gylcol | 0.5 |
| Polyquaternium-87 | 0.6 |
| Polyquaternium-2 | 0.6 |
| Amodimethicone | 0.2 |
| Coenzyme Q10 | 0.05 |
| Oleic acid | 0.05 |
| Perfume | 0.4 |
| Ammonia, 25% | ad pH 9.8 |
| Water | ad 100.0 |

The permanent wave achieved with this composition was similar to the one obtained with the composition according to Example 1.

Exclusion of the Polyquaternium-87 and Polyquaternium-2 led to substantially weaker waves.

### Example 5:

A permanent waving product consisting of two Compositions A and B, filled into a two-chamber packaging the chambers of which were kept separate until application, was prepared by destruction of the separating wall and applied onto human hair rolled onto curlers. The hair was rinsed after about fifteen minutes processing and neutralized for about five minutes with a 2.5 % H₂O₂ neutralizer composition, rinsed again, shampooed and dried.

An expressive, even, intensive permanent wave was obtained.

An identical treatment which had no Polyquaternium-87 and Polyquaternium-2 showed a visibly inferior wave.

### Composition A:

| | |
|---|---|
| Ammonium hydrogen carbonate | 4.5 (g) |
| Polyquaternium-87 | 0.5 |
| Polyquaternium-2 | 0.5 |
| PEG-65-Hydrogenated castor oil | 0.8 |
| Isopropyl alcohol | 1.5 |
| Ethoxydiglycol | 2.0 |
| Cocoamidopropyl betaine | 1.0 |
| Perfume | 0.3 |
| Coenzyme Q10 | 0.05 |
| Turbidifying agent | 0.5 |
| Ammonia, 25% | ad pH 8.4 |
| Water | ad 72.0 |

### Composition B:

| | |
|---|---|
| Ammonium thioglycolate, 70% | 18.0 (g) |
| Thiolactic acid | 2.0 |
| 2-Methyl-1.3-propanediol | 0.5 |
| Ammonia, 25% | ad pH 5.5 |
| Water | ad 28.0 |

After mixing of both Compositions, a ready-to-use product with a pH-value of 7.4 was obtained.

### Example 6:

A permanent waving product filled into a two-chamber package was prepared in analogy to Example 4:

### Composition A:

| | |
|---|---|
| Ammonium hydrogen carbonate | 3.5 (g) |
| Polyquaternium-87 | 0.5 |
| Polyquaternium-2 | 0.5 |
| Ethanol | 0.5 |
| 1-Methoxypropanol | 1.0 |
| Cocoamidopropyl betaine | 1.0 |
| PEG-25-glyceryl cocoate | 0.8 |
| Coenzyme Q10 | 0.1 |
| Oleic acid | 0.05 |
| Perfume | 0.3 |
| Turbidifying agent | 0.5 |
| Ammonia, 25% | ad pH 8.3 |
| Water | ad 72.0 |

### Composition B:

| | |
|---|---|
| Ammonium thioglycolate, 70% | 13.0 (g) |
| Thiolactic acid | 0.5 |
| 2-Methyl-1.3-propanediol | 1.5 |
| Ammonia, 25% | ad pH 5.5 |
| Water | ad 28.0 |

A product with a pH-value of 7.4 was obtained by mixing of the compositions immediately prior to application. After application onto dyed hair as described in Example 3, this mixture resulted in an expressive permanent wave, which had not effect whatever on the color gloss and color intensity.

### Example 7:

### Alkaline Permanent Waving Gel

| | |
|---|---|
| Ammonium thioglycolate, 70% | 15.0 (g) |
| Ammonium hydrogen carbonate | 4.5 |
| PEG-40-Hydrogenated castor oil | 0.7 |
| C₁₂-C₁₈-Fatty alcohol mixture | 3.5 |
| Cetrimonium chloride | 2.0 |
| Amodimethicone | 0.05 |
| 2-Methyl-1.3-propanediol | 0.5 |
| Polyquaternium-87 | 0.45 |
| Polyquaternium-2 | 0.45 |
| Coenzyme Q10 | 0.1 |
| Perfume | 0.3 |
| Ammonia, 25% | ad pH 8.0 |
| Water | ad 100.0 |

### Intermediate treatment composition

| | |
|---|---|
| Asparagic acid | 0.25% by weight |
| Glutamic acid | 0.50 |
| Alanin DL | 0.25 |
| Magnesium sulfate | 10.00 |
| Polyquaternium-87 | 0.25 |
| Water | q.s. to 100 |

The above composition had a pH of 4.10.

### Example 8:

### Straightening Composition

| Thioglycolic acid | 8.0 (% by wt.) |
|---|---|
| C₁₆-C₂₂-Fatty alcohol mixture | 3.5 |
| Oleth-50 | 2.5 |
| Laureth-23 | 1.5 |
| Polyquaternium-87 | 0.5 |
| Polyquaternium-2 | 0.5 |
| Ethanol | 5.0 |
| Perfume | 0.6 |
| Monoethanolamine | ad pH 9.3 |
| Water | ad 100.0 |

This composition constitutes an effecting smoothing composition for kinky hair.

## Claims

1. Composition for the permanent shaping of human hair, **characterized in that** it comprises at least one reducing agent, a first cationic polymer consisting of copolymer of vinylpyrrolidone and vinylimidazole and a homopolymer of dimethyl diallyl ammonium chloride and a second cationic polymer selected from urea copolymers.

2. Composition according to claim 1 **characterized in that** the reducing agents are selected from thioglycolic acid, thiolactic acid and their salts, cystein and its hydrochloride salt, acetylcystein.

3. Composition according to claims 1 and 2 **characterized in that** it comprises reducing agent at a concentration of 0.5 to 15% by weight, calculated to total composition.

4. Composition according to any of the preceding claims **characterized in that** the first cationic polymer is Polyquaternium-87 and it has a cationic charge density of between 3 and 4 meq/g at pH 7.0.

5. Composition according to any of the preceding claims **characterized in that** the second cationic polymer is Polyquaternium-2.

6. Composition according to any of the preceding claims **characterized in that** the first polymer is comprised at a concentration of 0.1 to 2.5%, preferably 0.2 to 2%, more preferably 0.25 to 1.5% and most preferably 0.25 to 1% by weight calculated to total composition.

7. Composition according to any of the preceding claims **characterized in that** the second polymer is comprised at a concentration of 0.1 to 2.5%, preferably 0.2 to 2%, more preferably 0.25 to 1.5% and most preferably 0.25 to 1% by weight calculated to total composition.

8. Composition according to any of the preceding claims **characterized in that** the first and the second cationic polymers are comprised at a total concentration in the range of 0.2 to 5%, preferably 0.2 to 2%, more preferably 0.25 to 1.5% and most preferably 0.25 to 1% by weight calculated to total composition, and preferably at a weight ratio of the first to the second cationic polymer in the range of 5:1 to 1:5, preferably 3:1 to 1:3 and more preferably 2:1 to 1:2 and most preferably 1:1.

9. Composition according to any of the preceding claims, **characterized in that** it has a Brookfield viscosity of 1 to 50,000 mPa.s at 20°C.

10. Composition according to any of the preceding claims **characterized in that** it comprises at least one surfactant selected from anionic, nonionic, cationic and amphoteric ones at a concentration of 0.05 to 10% by weight, calculated to total composition.

11. Composition according to any of the preceding claims **characterized in that** it comprises at least one fatty alcohol with a chain length of 12 to 24 C atoms and/or least one organic solvent and/or at least one ubichinone of the formula 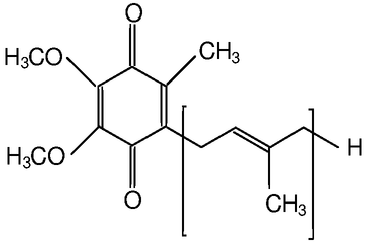 where n is a number between 1 and 10, and/or at least one silicone compound, preferably aminated silicone and/or at least one thickening agent.

12. Composition according to any of the preceding claims **characterized in that** it has a pH in the range of 6.5 to 10.5.

13. Process for permanent waving hair **characterized in that** hair is washed or shampooed first and wound on curlers and subsequently a composition according to claims 1 to 11 is applied onto hair and at the end of the 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair.

14. Process according to claim 13 **characterized in that** after rinsing off the reducing agent from hair, an intermediate treatment composition optionally comprising a first cationic polymer consisting of a copolymer of vinylpyrrolidone and vinylimidazole and a homopolymer of dimethyl diallyl ammonium chloride and a second cationic polymer selected from urea copolymers is applied onto hair and without rinsing off and after removal of the excess amount with towel, an oxidizing composition is applied and at the end of the processing they are rinsed off from hair.

15. Process for straightening hair **characterized in that** hair is washed and/or shampooed and dried and reducing composition according to claims 1 to 11 is applied onto dry hair and processed for 5 to 60 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

## Patentansprüche

1. Zusammensetzung zum dauerhaften Formen von menschlichem Haar, **dadurch gekennzeichnet, dass** sie mindestens ein Reduktionsmittel, ein erstes kationisches Polymer, das aus einem Copolymer aus Vinylpyrrolidon und Vinylimidazol und einem Homopolymer aus Dimethyldiallylammoniumchlorid besteht, und ein zweites kationisches Polymer aufweist, das aus Harnstoff-Copolymeren ausgewählt ist.

2. Zusammensetzung nach Anspruch 1, **dadurch gekennzeichnet, dass** die Reduktionsmittel aus Thioglykolsäure, Thiomilchsäure und deren Salzen, Cystein und seinem Hydrochloridsalz, Acetylcystein ausgewählt sind.

3. Zusammensetzung nach Anspruch 1 und 2, **dadurch gekennzeichnet, dass** sie Reduktionsmittel in einer Konzentration von 0,5 Gewichts-% bis 15 Gewichts-% aufweist, bezogen auf die Gesamtzusammensetzung.

4. Zusammensetzung nach einem der vorhergehenden Ansprüche, **dadurch gekennzeichnet, dass** das erste kationische Polymer Polyquaternium-87 ist und es eine kationische Ladungsdichte von 3 meq/g bis 4 meq/g bei pH 7,0 aufweist.

5. Zusammensetzung nach einem der vorhergehenden Ansprüche, **dadurch gekennzeichnet, dass** das zweite kationische Polymer Polyquaternium-2 ist.

6. Zusammensetzung nach einem der vorhergehenden Ansprüche, **dadurch gekennzeichnet, dass** das erste Polymer in einer Konzentration von 0,1 Gewichts-% bis 2,5 Gewichts-%, vorzugsweise 0,2 Gewichts-% bis 2 Gewichts-%, insbesondere 0,25 Gewichts-% bis 1,5 Gewichts-% und am besten 0,25 Gewichts-% bis 1 Gewichts-% enthalten ist, bezogen auf die Gesamtzusammensetzung.

7. Zusammensetzung nach einem der vorhergehenden Ansprüche, **dadurch gekennzeichnet, dass** das zweite Polymer in einer Konzentration von 0,1 Gewichts-% bis 2,5 Gewichts-%, vorzugsweise 0,2 Gewichts-% bis 2 Gewichts-%, insbesondere 0,25 Gewichts-% bis 1,5 Gewichts-% und am besten 0,25 Gewichts-% bis 1 Gewichts-% enthalten ist, bezogen auf die Gesamtzusammensetzung.

8. Zusammensetzung nach einem der vorhergehenden Ansprüche, **dadurch gekennzeichnet, dass** das erste und das zweite kationische Polymer in einer Gesamtkonzentration im Bereich von 0,2 Gewichts-% bis 5 Gewichts-%, vorzugsweise 0,2 Gewichts-% bis 2 Gewichts-%, insbesondere 0,25 Gewichts-% bis 1,5 Gewichts-% und am besten 0,25 Gewichts-% bis 1 Gewichts-%, bezogen auf die Gesamtzusammensetzung, und vorzugsweise in einem Gewichtsverhältnis des ersten zum zweiten kationischen Polymer im Bereich von 5:1 bis 1:5, vorzugsweise 3:1 bis 1:3 und insbesondere 2:1 bis 1:2 und am besten 1:1 enthalten sind.

9. Zusammensetzung nach einem der vorhergehenden Ansprüche, **dadurch gekennzeichnet, dass** sie eine Brookfield-Viskosität von 1 mPa s bis 50.000 mPa s bei 20 °C aufweist.

10. Zusammensetzung nach einem der vorhergehenden Ansprüche, **dadurch gekennzeichnet, dass** sie mindestens ein Tensid, ausgewählt aus anionischen, nichtionischen, kationischen und amphoteren, in einer Konzentration von 0,05 Gewichts-% bis 10 Gewichts-% aufweist, bezogen auf die Gesamtzusammensetzung.

11. Zusammensetzung nach einem der vorhergehenden Ansprüche, **dadurch gekennzeichnet, dass** sie mindestens einen Fettalkohol mit einer Kettenlänge von 12 bis 24 C-Atomen und/oder mindestens ein organisches Lösungsmittel und/oder mindestens ein Ubichinon der Formel 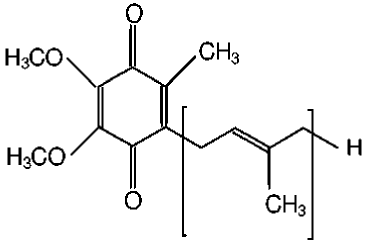 wobei n eine Zahl von 1 bis 10 ist, und/oder mindestens eine Silikonverbindung, vorzugsweise aminiertes Silikon, und/oder mindestens ein Verdickungsmittel aufweist.

12. Zusammensetzung nach einem der vorhergehenden Ansprüche, **dadurch gekennzeichnet, dass** sie einen pH-Wert im Bereich von 6,5 bis 10,5 aufweist.

13. Verfahren zum permanenten Wellen von Haar, **dadurch gekennzeichnet, dass** das Haar zuerst gewaschen oder shampooniert wird und auf Lockenwickler gezogen wird und anschließend eine Zusammensetzung nach Anspruch 1 bis 11 auf das Haar aufgebracht wird und am Ende der Einwirkzeit von 1 min bis 45 min, vorzugsweise 1 min bis 30 min, abhängend von der Haarstärke, mit Leitungswasser aus dem Haar ausgespült wird und eine oxidierende Zusammensetzung, die mindestens ein Oxidationsmittel aufweist, vorzugsweise Wasserstoffperoxid oder Natriumbromat in einer Konzentration von 0,5 Gewichts-% bis 10 Gewichts-%, bezogen auf die gesamte oxidierende Zusammensetzung, auf das Haar aufgebracht wird und 1 min bis 20 min lang auf dem Haar belassen wird und ausgespült wird und die Lockenwickler aus dem Haar entfernt werden.

14. Verfahren nach Anspruch 13, **dadurch gekennzeichnet, dass** nach dem Ausspülen des Reduktionsmittels aus dem Haar eine Zwischenbehandlungszusammensetzung auf das Haar aufgebracht wird, die gegebenenfalls ein erstes kationisches Polymer, das aus einem Copolymer aus Vinylpyrrolidon und Vinylimidazol und einem Homopolymer aus Dimethyldiallylammoniumchlorid besteht, und ein zweites kationisches Polymer aufweist, das aus Harnstoff-Copolymeren ausgewählt ist, und ohne Ausspülen und nach dem Entfernen der überschüssigen Menge mit einem Handtuch eine oxidierende Zusammensetzung aufgebracht wird und sie am Ende des Einwirkens aus dem Haar ausgespült werden.

15. Verfahren zum Glätten von Haar, **dadurch gekennzeichnet, dass** das Haar gewaschen und/oder shampooniert und getrocknet wird und eine reduzierende Zusammensetzung nach Anspruch 1 bis 11 auf das trockene Haar aufgebracht wird und 5 min bis 60 min einwirkt und mit Wasser ausgespült und getrocknet wird und das trockene Haar bei einer Temperatur von 130 °C bis 210 °C physikalisch mit Glätteisen geglättet wird und anschließend eine oxidierende Zusammensetzung, die mindestens ein Oxidationsmittel, vorzugsweise Wasserstoffperoxid oder Natriumbromat, in einer Konzentration von 0,5 Gewichts-% bis 10 Gewichts-%, bezogen auf die gesamte oxidierende Zusammensetzung, aufweist, auf das Haar aufgebracht wird und 1 min bis 20 min lang auf dem Haar belassen wird und ausgespült wird.

## Revendications

1. Composition pour le façonnage permanent de cheveux humains, **caractérisée en ce qu'**elle comprend au moins un agent réducteur, un premier polymère cationique consistant en un copolymère de vinylpyrrolidone et de vinylimidazole et en un homopolymère de chlorure de diméthyl diallyl ammonium, et un second polymère cationique choisi parmi des copolymères d'urée.

2. Composition selon la revendication 1, **caractérisée en ce que** les agents réducteurs sont choisis parmi l'acide thioglycolique, l'acide thiolactique et leurs sels, la cystéine et son sel chlorure, l'acétylcystéine.

3. Composition selon les revendications 1 et 2, **caractérisée en ce qu'**elle comprend un agent réducteur à une concentration de 0,5 à 15 % en poids, calculée par rapport à la composition totale.

4. Composition selon l'une quelconque des revendications précédentes, **caractérisée en ce que** le premier polymère cationique est du Polyquaternium-87 et a une densité de charge cationique entre 3 et 4 meq/g à pH 7,0.

5. Composition selon l'une quelconque des revendications précédentes, **caractérisée en ce que** le second polymère cationique est du Polyquaternium-2.

6. Composition selon l'une quelconque des revendications précédentes, **caractérisée en ce que** le premier polymère est contenu à une concentration de 0,1 à 2,5 %, de préférence de 0,2 à 2 %, plus préférentiellement de 0,25 à 1,5 % et idéalement de 0,25 à 1 % en poids calculée par rapport à la composition totale.

7. Composition selon l'une quelconque des revendications précédentes, **caractérisée en ce que** le second polymère est contenu dans une concentration de 0,1 à 2,5 %, de préférence de 0,2 à 2 %, plus préférentiellement de 0,25 à 1,5 % et idéalement de 0,25 à 1 % en poids, calculée par rapport à la composition totale.

8. Composition selon l'une quelconque des revendications précédentes, **caractérisée en ce que** le premier et le second polymère cationiques sont contenus dans une concentration totale dans la plage de 0,2 à 5 %, de préférence de 0,2 à 2 %, plus préférentiellement de 0,25 à 1,5 % et idéalement de 0,25 à 1 % en poids, calculée par rapport à la composition totale, et de préférence avec un rapport de poids du premier polymère cationique par rapport au second dans la plage de 5 : 1 à 1 : 5, de préférence de 3 : 1 à 1 : 3 et plus préférentiellement de 2 : 1 à 1 : 2 et idéalement de 1 : 1.

9. Composition selon l'une quelconque des revendications précédentes, **caractérisée en ce qu'**elle a une viscosité de Brookfield de1 à 50 000 mPa à 20 °C.

10. Composition selon l'une quelconque des revendications précédentes, **caractérisée en ce qu'**elle comprend au moins un tensioactif choisi parmi les anioniques, les non ioniques, les cationiques et les amphotères à une concentration de 0,05 à 10 % en poids, calculée par rapport à la composition totale.

11. Composition selon l'une quelconque des revendications précédentes, **caractérisée en ce qu'**elle comprend au moins un alcool gras avec une longueur de chaîne de 12 à 24 atomes C et/ou au moins un solvant organique, et/ou au moins une ubiquinone selon la formule 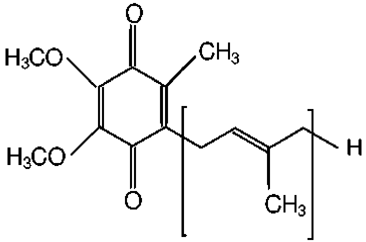 où n est un nombre entre 1 et 10, et/ou au moins un composé de silicone, de préférence un silicone aminé, et/ou au moins un agent épaississant.

12. Composition selon l'une quelconque des revendications précédentes, **caractérisée en ce qu'**elle a un pH dans la gamme de 6,5 à 10,5.

13. Procédé pour faire des ondulations permanentes des cheveux **caractérisé en ce que** les cheveux sont lavés ou shampouinés en premier lieu et enroulés sur des bigoudis, et qu'ensuite une composition selon les revendications 1 à 11 est appliquée sur les cheveux, à la fin d'une durée de traitement de 1 à 45 minutes, de préférence de 1 à 30 minutes, en fonction de la résistance des cheveux, est enlevée des cheveux par rinçage avec de l'eau du robinet et une composition oxydante comprenant au moins un agent oxydant, de préférence du peroxyde d'hydrogène ou du bromate de sodium à une concentration de 0,5 à 10 % en poids, calculée par rapport au total de la composition oxydante, est appliquée sur les cheveux et est laissée sur les cheveux de 1 à 20 minutes, et est enlevée par rinçage et les bigoudis sont enlevés des cheveux.

14. Procédé selon la revendication 13, **caractérisé en ce qu'**après l'enlèvement par rinçage de l'agent réducteur des cheveux, une composition de traitement intermédiaire comprenant un premier polymère cationique, constitué d'un copolymère de vinylpyrrolidone et de vinyl imidazole et un homopolymère de chlorure de diméthyl diallyl ammonium et un second polymère cationique choisi parmi les copolymères d'urée est éventuellement appliquée sur les cheveux et sans enlèvement par rinçage, et après l'enlèvement de la quantité en excès avec une serviette, une composition oxydante est appliquée, et à la fin du traitement, elles sont enlevées des cheveux par rinçage.

15. Procédé pour le défrisage des cheveux **caractérisé en ce que** les cheveux sont lavés et/ou shampouinés, et séchés, et une composition réductrice selon les revendications 1 à 11 est appliqué sur les cheveux secs et est laissée en traitement pendant 5 à 60 minutes, et est enlevée par rinçage avec de l'eau, et les cheveux sont séchés, et physiquement défrisés au fer chaud à une température de 130 à 210 °C et ensuite une composition oxydante comprenant au moins un agent oxydant, de préférence du peroxyde d'hydrogène ou du bromate de sodium à une concentration de 0,5 à 10 % en poids calculée par rapport à la composition oxydante totale, est appliquée sur les cheveux et laissée sur les cheveux 1 à 20 minutes, et enlevée par rinçage.
